# EUROPÄISCHE PATENTANMELDUNG

(11) **EP 4 285 952 A1**
(43) Veröffentlichungstag der Anmeldung: **06.12.2023**
(21) Anmeldenummer: 22176849.2
(22) Anmeldetag: 01.06.2022
(51) Int. Cl.: A61M 1/00, A61C 17/08

(54) **SPEICHELSAUGER**

(71) Anmelder: Ivoclar Vivadent AG, 9494 Schaan (LI)
(72) Erfinder: Lichtensteiger, Markus, 9462 Montlingen (CH)
(74) Vertreter: Baldus, Oliver

(57) **Zusammenfassung**

Es ist ein Speichelsauger, mit einem Kunststoff-Formteil aus Spritzguss, das eine Kupplung für den Anschluss an einen Saugschlauch, ggf. über einen Adapter, ausgebildet und auf den Saugschlauch aufgesteckt oder auf andere Art mit dem Saugschlauch verbunden ist und insbesondere ein Zungenschild ausbildet, vorgesehen. Durch das Formteil verläuft ein erster Saugkanal. Auf das Kunststoff-Formteil ist ein massiver, also unporöser, Weichkörper aufgesteckt oder angesteckt und formschlüssig mit dem Formteil verbunden, durch welchen Weichkörper ein zweiter Saugkanal verläuft, der gegenüber dem ersten Saugkanal höhenversetzt ist. Insbesondere an der Seite des zweiten Saugkanals, die dem ersten Saugkanal gegenüberliegt, ist in dem Weichkörper ein Saugbereich mit mindestens einer Saugöffnung für Speichel ausgebildet.

## Beschreibung

Die Erfindung betrifft einen Speichelsauger, gemäß dem Oberbegriff von Anspruch 1.

Speichelsauger sind seit langem bekannt. Sie dienen dazu, Speichel, der sich im Mund eines Patienten ansammelt, zu entfernen und dem Zahnarzt ein störungsfreies Arbeiten zu ermöglichen.

Hierzu dient ein Saugschlauch. Der Saugschlauch kann entweder unter der Zunge des Patienten enden und über seine Saugöffnung den Speichel ansaugen. Alternativ kann der Saugschlauch auch mit einem Kunststoff-Formteil ausgerüstet sein, durch das hindurch das Absaugen erfolgt.

Wenn ein Kunststoff-Formteil verwendet wird, kann dieses auch mit einem Zungenschutz verbunden sein oder diesen ausbilden. Der Zungenschutz dient dazu, die Zunge des Patienten vom Behandlungsort fernzuhalten.

Eine solche Lösung ist z.B. aus der US 4 906 188 A1 bekannt. Der dortige Zungenschutz weist in seinem unteren Bereich Saugkanäle auf, die der Abfuhr von Speichel dienen sollen. Der untere Bereich ist einstückig mit dem oberen Bereich, dem eigentlichen Zungenschutz, verbunden.

Diese Lösung hat den folgenden Nachteil: Die Zunge eines Patienten neigt dazu, sich zu bewegen, gleichermaßen, zu "spielen". Wenn die Zunge des Patienten den oberen Teil des Zungenschutzes bewegt, bewegt sich auch der untere Teil des Zungenschutzes mit. Der untere Teil ruht jedoch in empfindlichem Weichgewebe unterhalb der Zunge.

Daher wird die genannte Lösung von den Patienten als unangenehm empfunden und hat eine geringe Akzeptanz.

Ferner ist es bekannt geworden, das Formteil mit einem Schaumstoff zu polstern. Der Schaumstoff oder Schwamm ist offenporig, um die erwünschte Saugwirkung bereitstellen zu können. Der Schwamm macht die Bewegung des unteren Teils erträglicher, nachdem er wesentlich nachgiebiger und weicher als das Formteil ist.

Die Saugwirkung ist bei dieser Lösung jedoch etwas geringer als bei der Lösung gemäß der vorstehenden US-Patentschrift. Dies liegt darin begründet, das im oberen Teil des Schwammes viel Luft angesaugt wird.

Dies liegt daran, dass der Schwamm multidirektional offenporig ist. Es ist also mit einem Schwamm nicht möglich, gezielt lediglich den unteren Bereich abzusaugen, also ohne, dass sich die Saugwirkung des Saugschlauchs durch den Schwamm hindurch vergleichmäßigt.

Um trotz des Schwammes eine bessere Absaugung zu gewährleisten, ist es auch bereits vorgeschlagen worden, den oberen Teil des den Saugschlauch umgebenden Schwammes kurzerhand abzudecken. Mit diesem Vorschlag lässt sich die Fehlluft zwar etwas reduzieren, jedoch gelangt Fehlluft aufgrund der multidirektionalen Offenporigkeit des Schwammes regelmäßig dennoch in den Saugschlauch.

Demgegenüber liegt der Erfindung die Aufgabe zugrunde, einen Speichelsauger gemäß dem Oberbegriff von Anspruch 1 zu schaffen, der einen guten Komfort mit einer guten Saugleistung verbindet.

Diese Aufgabe wird erfindungsgemäß durch Anspruch 1 gelöst. Vorteilhafte Weiterbildungen ergeben sich aus den Unteransprüchen.

Erfindungsgemäß weist der Speichelsauger ein Kunststoff-Formteil aus Spritzguss auf. Das Formteil hat eine Kupplung für den Anschluss an einen Saugschlauch, ggf. über einen Adapter. Es ist auf den Saugschlauch aufgesteckt oder auf andere Art mit dem Saugschlauch verbunden und bildet insbesondere ein Zungenschild aus. Durch das Formteil verläuft ein erster Saugkanal.

An der der Kupplung gegenüberliegenden Seite ist auf das Kunststoff-Formteil ein massiver, also unporöser, Weichkörper aufgesteckt oder angesteckt. Der Weichkörper besteht bevorzugt aus einem Weichkunststoff wie einem Elastomer. Der Weichkörper ist bevorzugt weicher als das Formteil. Der Weichkörper ist bevorzugt formschlüssig mit dem Formteil verbunden.

Der Weichkörper ist porenfrei, weist also kein Poren auf, die sich typischerweise stochastisch verteilt - wie bei einem Schwamm - durch einen Körper erstrecken würden. Vielmehr ist er - außer an den Stellen, an denen gezielt Saugöffnungen ausgebildet sind - undurchlässig.

Wenn an den Saugschlauch Unterdruck angelegt wird, wird dieser durch den erfindungsgemäßen Speichelsauger ausschließlich zu den Saugöffnungen transferiert. Fehlluft wird vermieden.

Durch den Weichkörper verläuft erfindungsgemäß ein zweiter Saugkanal, der gegenüber dem ersten Saugkanal höhenversetzt ist. Damit ist gemeint, dass die Saugkanäle sich im wesentlichen in die gleiche Richtung erstrecken, aber nicht koaxial, sondern in der Richtung quer zu Ihren Achsen versetzt zueinander. Bevorzugt ist der zweite Saugkanal weiter vom Zungenschild entfernt als der erste Saugkanal.

Der erste Saugkanal erstreckt sich bevorzugt in der Verlängerung der Kupplung und damit des Saugschlauchs. Er ist deutlich kürzer als der zweite Saugkanal, z.B. 5mm bis 20m lang. Der zweite Saugkanal ist z.B. 15mm bis 50mm lang.

Zwischen den Saugkanäle nist bevorzugt ein Übergangsbereich ausgebildet. Dieser stellt sich als ein Hohlraum dar, der sich von dem ersten zu dem zweiten Suagkanal erstreckt, mit einer Ausrichtung schräg zu den Achsen der beiden Saugkanäle und die beiden Saugkanäle verbindend. Der Übergangsbereich hat bevorzugt einen größeren Strömungsquerschnitt als der erste Saugkanal und als der zweite Saugkanal. Er ist bevorzugt an einer Stelle ausgebildet, an der der Weichkörper formschlüssig mit dem Formteil verbunden ist.

Der Strömungsquerschnitt des ersten Saugkanals ist bevorzugt größer als der des Saugschlauches, z.B. um 20% bis 200%. Auch der Strömungsquerschnitt des zweiten Saugkanals ist bevorzugt größer als der des Saugschlauches, z.B. um 40% bis 400%. Die Strömungsquerschnitte beider Saugkanäle sind bevorzugt höher als breit und besonders bevorzugt hochoval.

Der Weichkörper und damit der zweite Saugkanal sind bevorzugt gebogen. Bevorzugt so, dass die Unterseite des Weichkörpers, also die vom Zungenschild abgewandte Seite, konvex und die Oberseite, also die dem Zungenschild benachbarte Seite, konkav ist. Dies erlaubt ein körperverträgliches Anschmiegen des Weichkörpers an den Zungengrund.

Erfindungsgemäß ist, insbesondere an der Seite des zweiten Saugkanals, die dem ersten Saugkanal gegenüberliegt, also der Unterseite des Weichkörpers, in dem Weichkörper ein Saugbereich mit mindestens einer Saugöffnung für Speichel ausgebildet. Diese Saugöffnung ist dafür bestimmt, der Speichel des Patienten, der sich am Zungengrund sammelt, abzusaugen.

Die mindestens eine Saugöffnung ist bevorzugt als Schlitz ausgebildet, der sich mit seiner Längserstreckung quer zum zweiten Saugkanal erstreckt, über die ganze Unterseite des Weichkörpers, und erst am Ende des unteren Drittels der Seitenwand des Weichkörpers endet.

Durch den Schlitz wird nicht nur eine Absaugung mit großem Strömungsquerschnitt - und damit geringer Strömungsgeschwindigkeit auch an dieser Stelle - ermöglicht. Zudem wird die Nachgiebigkeit des Weichkörpers gegen ein Verbiegen um eine Achse quer zur Erstreckung des zweiten Saugkanals deutlich erhöht, da durch den Schlitz der Weichkörper an dieser Stelle weniger Material hat.

Bevorzugt ist eine Mehrzahl gleichmäßig über die Unterseite des Weichkörpers verteilter Schlitze vorgesehen, z.B. 3 bis 10 Schlitze, besonders bevorzugt 5 bis 7 Schlitze. Diese haben je die beschriebene Doppelfunktion "Materialschwächung" und "Strömungsgeschwindigkeitsabsenkung". Wenn der Speichelsauger z.B. so in den Mund des Patienten eingelegt wird, dass er am Kieferkamm anliegt, biegt er sich an dem Kontaktbereich zum Kieferkamm kurzerhand nach oben, so dass der Speichelsauger trotz der dadurch begünstigen Neigung, nach oben zu wandern, am Zungengrund verbleibt.

Erfindungsgemäß ist es günstig, wenn der Weichkörper formschlüssig mit dem Formteil verbunden ist. Der Formschluss stellt sicher, dass sich der Weichkörper nicht versehentlich im Mund des Patienten von dem Formteil löst. Anstelle dessen ist es auch möglich, den den Weichkörper durch Kleben auf dem Formteil zu befestigen.

In einer bevorzugten Ausgestaltung des Weichkörpers sind an seiner Unterseite Rippen vorgesehen, die sich je zwischen den Schlitzen erstrecken. Die Rippen erstrecken sich wie die Schlitze senkrecht zu dem zweiten Saugkanal. Sie dienen als eine Art Abstandshalter und verhindern, dass die benachbarte Saugöffnung sich durch den Saugunterdruck an dem Weichgewebe festsaugt und damit keine Saugfunktion mehr hat.

In vorteilhafter Ausgestaltung sind ferner in der Verlängerung der Schlitze je kleine Saugöffnungen vorgesehen. Diese dienen der zusätzlichen Absaugung von Speichel, wenn der Weichkörper seitlich am Kieferkamm anliegt.

Weitere Einzelheiten, Vorteile und Merkmale der Erfindung ergeben sich aus der nachfolgenden Beschreibung eines Ausführungsbeispiels der Erfindung anhand der Zeichnung.

Es zeigen:
Fig. 1 eine Seitenansicht einer Ausführungsform eines erfindungsgemäßen Speichelsaugers; und
Fig. 2 die Ausführungsform gemäß Fig. 1, jedoch in perspektivischer Darstellung.

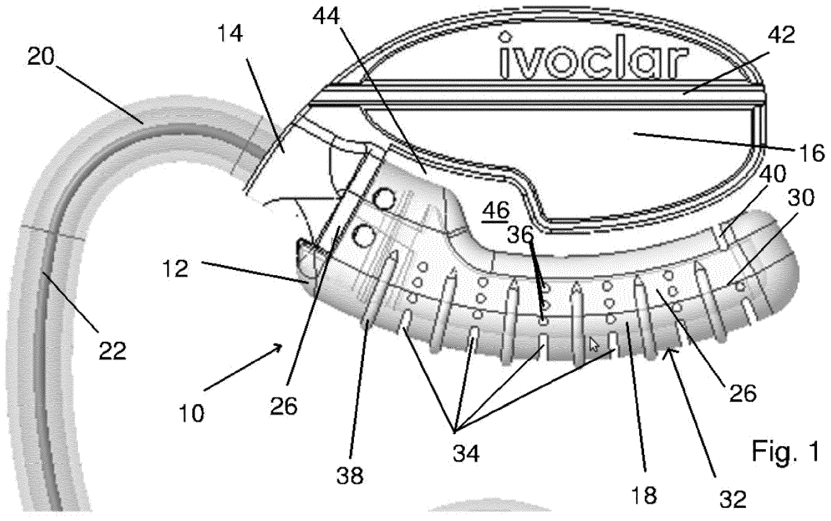

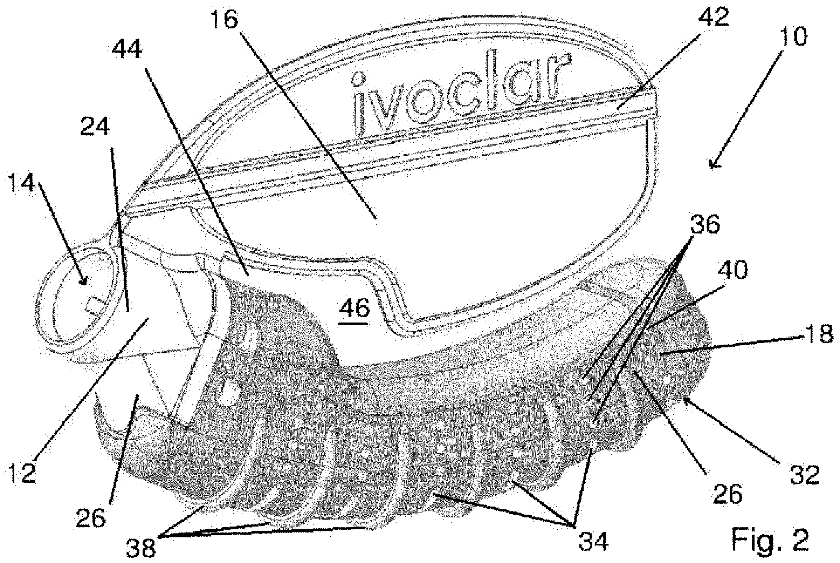

In Fig. 1 und 2 ist je ein erfindunsgemäßer Speichelsauger 10 dargestellt. In beiden Figuren werden die gleichen Bezugszeichen verwendet. Der Speichelsauger 10 besteht aus einem Kunststoff-Formteil 12, zu dem auch eine Kupplung 14 gehört, einem Zungenschild 16 und einem Weichkörper 18. Die Kupplung 14 ist für den Anschluss an einen Saugschlauch 20 bestimmt. Der Saugschlauch 20 ist in die Kupplung 14 einsteckbar und nach dem Einstecken fest mit dieser verbunden. Bevorzugt verläuft in dem Saugschlauch ein plastisch verformbare Draht 22. Dieser kann entweder lose in oder an dem Formteil 12 enden oder in dieses eingesteckt sein und dort verankert sein. Der Draht 22 dient in an sich bekannter Weise der Aussteifung des Saugschlauchs 20.

Zungenschild 16 und Formteile 12 sind einstückig zueinander ausgebildet. Sie bestehen aus Kunststoff und sind bevorzugt durch Spritzguss hergestellt.

In dem Formteil 12 ist ein erster Saugkanal 24 in Verlängerung der Kupplung 14 ausgebildet. Der Saugkanal 24 verläuft insofern im Wesentlichen entlang der Längserstreckung des Zungenschilds 16.

Zur der Kupplung 14 gegenüberliegenden Seite hin ist der erste Saugkanal 24 erweitert, und zwar nach unten hin, also zu der vom Zungenschild 16 abgewandten Seite hin. Dort bildet das Formteil 12 einen Übergangsbereich 26 zum Weichkörper 18 und zu dem zweiten Saugkanal 28.

Beide Saugkanäle erstrecken sich im Übergangsbereich 26 parallel zueinander, jedoch höhenversetzt, nämlich so, dass der zweite Saugkanal 28 vom Zungenschild 16 weiter entfernt ist als der erste Saugkanal 24. Im weiteren Verlauf ist der Weichkörper 18 und damit der zweite Saugkanal 28 gebogen um einen Biegeradius, der sich in der Darstellung gemäß Figur 1 nach oben erstreckt und oberhalb des Zungenschildes 16 endet.

Eine Achse 30 des zweiten Saugkanals verläuft also gebogen und zwar, bezogen auf einen geraden Achsenverlauf, vom Übergangsbereich 26 ausgehend nach oben gebogen.

An einer Unterseite 32 des Weichkörpers 18, also der Seite, die dem Zungenschild 16 gegenüberliegt, sind eine Vielzahl von Schlitzen 34 in dem Weichkörper 18 ausgebildet. Die Schlitze erstrecken sich über die gesamte Unterseite des Weichkörper 18 und auch in die Seitenwand des Weichkörpers 18 hinein.

Die Schlitze haben bei dem hier dargestellten Ausführungsbeispiel eine Breite von 0,5 mm und eine Länge von 10 mm, gemessen an der Außenseite des Weichkörpers. Der Weichkörper 18 ist an seiner Unterseite 32 bidirektional konvex. Er ist auch mit einem Krümmungsradius, dessen Achse sich entlang der Achse 30 erstreckt, gebogen. Der Krümmungsradius ist damit wesentlich geringer als der oben beschriebene Biegeradius.

In der Verlängerung der Schlitze 34 sind zusätzlich eine Mehrzahl von Kleinsaugöffnungen 36 vorgesehen. In dem dargestellten Ausführungsbeispiel sind je drei Kleinsaugöffnungen 36 in jeder Seitenwand des Weichkörpers 18 ausgebildet. Die Kleinsaugöffnungen 36 haben einen Durchmesser von ebenfalls etwa 0,5 mm.

An der Unterseite 32 des Weichkörpers 18 erstrecken sich parallel zu den Schlitzen 34 Rippen 38 aus der Unterseite 32 heraus. Diese enden an der Seitenwand des Weichkörpers 18, etwa in der Höhe, in der auch die Kleinsaugöffnungen 36 enden. Damit werden sowohl diese als auch die Schlitze 34 von der Anlage an Weichgewebe abgehalten, so dass kein Selbstverschließen zu befürchten ist.

Die Schlitze 34 und die Rippen 38 erstrecken sich je parallel zueinander und zwar in einem gleichmäßigen Raster. Im dargestellten Ausführungsbeispiel sind an der Unterseite 32 6 Schlitze 34 und 6 Rippen 38 ausgebildet. Es versteht sich, dass diese Anzahl je in weiten Bereichen an die Erfordernisse anpassbar ist.

Der Weichkörper 18 ist an seiner dem Formteil 12 gegenüberliegenden Seite geschlossen. Dort bildet er ein distales Ende. An dem distalen Ende ist an der Oberseite des Weichkörpers 18 ein Zusatzschlitz 40 ausgebildet, dem eine besondere Funktion zukommt. Der Zusatzschlitz 40 kommt dann in Funktion, wenn der Patient im Zahnarztstuhl in eine starke Rückenlage abgesenkt wird. In dieser Lage ist das distale Ende des Weichkörpers 18 der tiefste Teil im Mund des Patienten. Sich ansammelnder Speichel soll dann von dem Zusatzschlitz 40 abgesaugt werden.

Der Schlitz 40 liegt zudem dem dem distalen Ende nächsten Schlitz 34 genau gegenüber. Beide Schlitze erstrecken sich bis deutlich in die Seitenwände des Weichkörpers 18 hinein. An dieser Stelle ist demnach die Materialschwächung am stärksten. Wenn der Zahnarzt es für sinnvoll hält, lässt sich daher das distale Ende des Weichkörpers ohne weiteres abtrennen, beispielsweise mittels einer Schere, eines Messers oder auch einfach mit der Hand. Der Weichkörper 18 hat dann ein offenes Ende, das auch in der Rückenlage des Patienten als Saugöffnung dienen kann.

Besonders günstig ist es auch, dass der erfindungsgemäße Weichkörper 18 durch die Anordnung der Schlitze beliebig abtrennbar ist. Beispielsweise kann die Abtrennung am drittletzten Schlitz 34 vorgenommen werden, um eine Kindergröße des erfindungsgemäßen Speichelsaugers 10 bereitzustellen. Bevorzugt wird dann der Zungenschild 16 in gleicher Weise gekürzt, wozu ebenfalls eine - in den Figuren - nicht dargestellte Sollbruchstelle ausgebildet sein kann.

Besonders günstig ist es, dass der Zungenschild 16 ein Filmscharnier 42 über seine gesamte Länge ausbildet. Der obere Teil des Zungenschildes lässt sich durch das Filmscharnier 42 ohne weiteres leicht seitlich abbiegen. Der Zungenschild 16 erhält dadurch eine ebenfalls etwas gebogene Ausgestaltung und passt sich besser der Unterseite der Zunge des Patienten an.

Der Zungenschild ist an seiner Unterseite gebogen, und zwar so, dass ein im Wesentlichen gleichmäßig breiter Schlitz 44 zum Weichkörper 18 verbleibt. Der Schlitz 44 kann beispielsweise eine Breite von 2 bis 3 mm haben. Damit kann die Zunge nicht in den Bereich zwischen Zungenschild 16 und Weichkörper 18 eindringen. Der Schlitz ist etwa in der Mitte mit einer Aussparung 46 erweitert. An dieser Stelle wird so Platz geschaffen, wenn der Weichkörper 18 von dem Formteil 12 abgezogen werden soll und ausgetauscht werden soll.

In vorteilhafter Ausgestaltung ist mindestens das Formteil 12 mit dem Zungenschild 16 autoklavierbar.

## Patentansprüche

1. Speichelsauger, mit einem Kunststoff-Formteil aus Spritzguss, das eine Kupplung für den Anschluss an einen Saugschlauch, ggf. über einen Adapter, ausgebildet und auf den Saugschlauch aufgesteckt oder auf andere Art mit dem Saugschlauch verbunden ist und insbesondere ein Zungenschild ausbildet, durch welches Formteil ein erster Saugkanal verläuft, **dadurch gekennzeichnet, dass** auf das Kunststoff-Formteil ein massiver, also unporöser, Weichkörper aufgesteckt oder angesteckt und formschlüssig mit dem Formteil verbunden ist, durch welchen Weichkörper ein zweiter Saugkanal verläuft, der gegenüber dem ersten Saugkanal höhenversetzt ist, und dass, insbesondere an der Seite des zweiten Saugkanals, die dem ersten Saugkanal gegenüberliegt, in dem Weichkörper ein Saugbereich mit mindestens einer Saugöffnung für Speichel ausgebildet ist.

2. Speichelsauger nach Anspruch 1, **dadurch gekennzeichnet, dass** der erste Saugkanal sich mit seiner ersten Achse koaxial zum Saugschlauch erstreckt und der zweite Saugkanal sich mit seiner - ggf. gebogenen - zweiten Achse in die Richtung versetzt erstreckt, die von dem Zungenschild weg weist.

3. Speichelsauger nach Anspruch 1 oder 2, **dadurch gekennzeichnet, dass** der zweite Saugkanal einen länglichen, insbesondere ovalen oder eiförmigen Querschnitt aufweist, bevorzugt mit einer Höhe, die mehr als das doppelte der Breite beträgt.

4. Speichelsauger nach einem der vorhergehenden Ansprüche, **dadurch gekennzeichnet, dass** der Weichkörper biegsam mit einem Elastizitätsmodul von weniger als 1 GPa, insbesondere von weniger als 100 Mpa, ist, und insbesondere durchschnittlich eine größere Wandstärke als das Formteil aufweist.

5. Speichelsauger nach einem der vorhergehenden Ansprüche, **dadurch gekennzeichnet, dass** der Weichkörper im entspannten Zustand gebogen ist und eine konvexe Seite und eine konkave Seite hat, insbesondere die konvexe Seite an der Unterseite und die konkave Seite an der Oberseite, und dass die Saugöffnungen oder die Mehrzahl der Saugöffnungen an der konvexen Seite ausgebildet sind.

6. Speichelsauger nach einem der vorhergehenden Ansprüche, **dadurch gekennzeichnet, dass** der gebogene Weichkörper an einer konvexen Seite Schlitze als Saugöffnungen aufweist, die quer zum Biegeradius des Weichkörpers verlaufen und sich insbesondere über die ganze, oder im wesentlichen ganze, konvexe Seite des Weichkörpers erstrecken.

7. Speichelsauger nach einem der vorhergehenden Ansprüche, **dadurch gekennzeichnet, dass** der gebogene Weichkörper an einer konvexen Seite Schlitze als Saugöffnungen aufweist, die durch Geradebiegen des Weichkörpers schließbar sind.

8. Speichelsauger nach einem der vorhergehenden Ansprüche, **dadurch gekennzeichnet, dass** der gebogene Weichkörper an einer Seite Schlitze als Saugöffnungen und in Verlängerung dieser eine Mehrzahl demgegenüber kleinere Saugöffnungen, insbesondere Löcher, aufweist, und dass insbesondere mindestens eine Anordnung aus einem Schlitz und mindestens einer der benachbarten kleineren Saugöffnungen eine Sollbruchstelle bildet.

9. Speichelsauger nach einem der vorhergehenden Ansprüche, **dadurch gekennzeichnet, dass** ein Zungenschild sich entlang einer konkaven Oberseite des gebogenen Weichkörpers und von diesem weniger als 8mm beabstandet erstreckt, und insbesondere dort eine, dem Verlauf des gebogenen Weichkörpers folgende, gebogene Kante aufweist, und dass der Weichkörper den Zungenschild an seinem distalen Ende überragt.

10. Speichelsauger nach einem der vorhergehenden Ansprüche, **dadurch gekennzeichnet, dass** der Weichkörper gegenüber dem Saug-Unterdruck des Speichelsaugers, insbesondere in Höhe von zwischen 80 mbar und 250 mbar, formstabil ist.

11. Speichelsauger nach einem der vorhergehenden Ansprüche, **dadurch gekennzeichnet, dass** der zweite Saugkanal eine allseits - abgesehen von den Saugöffnungen und einer Auslassöffnung zum Formteil hin - geschlossene Hohlkammer bildet, die im Betrieb des Speichelsaugers unter Unterdruck steht.

12. Speichelsauger nach einem der vorhergehenden Ansprüche, **dadurch gekennzeichnet, dass** der erste und/oder der zweite Saugkanal einen gegenüber dem Saugschlauch größeren Querschnitt aufweist, und insbesondere einen Querschnitt, der gegenüber einem Kreisquerschnitt in Richtung des Zungenschildes vergrößert ist.

13. Speichelsauger nach einem der vorhergehenden Ansprüche, **dadurch gekennzeichnet, dass** ein Zungenschild sich in Verlängerung eines hochovalen oder elliptischen Querschnitts des zweiten Saugkanals erstreckt.

14. Speichelsauger nach einem der vorhergehenden Ansprüche, **dadurch gekennzeichnet, dass** der zweite Saugkanal einen über seine Längserstreckung bis zu einer Auslassöffnung zum Formteil hin im wesentlichen konstanten Querschnitt aufweist, welcher Querschnitt insbesondere größer als der freie Strömungsquerschnitt des Saugschlauchs ist.

15. Speichelsauger nach einem der vorhergehenden Ansprüche, **dadurch gekennzeichnet, dass** der Weichkörper an seiner dem Zungenschild gegenüberliegenden Seite eine Profilstruktur, insbesondere mit Rippen oder Vorsprüngen aufweist, mit einer über den Verlauf der Seite im wesentlichen gleichen Profilhöhe.

16. Speichelsauger nach Anspruch 15, **dadurch gekennzeichnet, dass** die Profilstruktur eine Höhe von mehr als 0,2 mm, insbesondere etwa 0,5mm, aufweist und die Saugöffnungen des Saugbereich am Grund der Profilstruktur enden, so dass insbesondere auch bei Anlage der Profilstruktur an Gewebe die Profilstruktur eine Blockade der Saugöffnung auch das Gewebe verhindert.

17. Speichelsauger nach einem der vorhergehenden Ansprüche, **dadurch gekennzeichnet, dass** das Formteils mit seiner Kupplung, insbesondere mit seinem Adapter, drehbar gegenüber dem Saugschlauch ist und insbesondere eine Querschnittsanpassung zwischen dem Saugschlauch und dem zweiten Saugkanal herstellt.

18. Speichelsauger nach einem der vorhergehenden Ansprüche, **dadurch gekennzeichnet, dass** der Saugschlauch eine integrierte Drahtaufnahme für die Aufnahme eines plastisch verformbaren Drahtes aufweist, die an dem Formteil, insbesondere für die Verbindung des Drahtes mit diesem, endet.

19. Speichelsauger nach einem der vorhergehenden Ansprüche, **dadurch gekennzeichnet, dass** der Weichkörper an seiner dem Zungenschild benachbarten Oberseite mindestens eine Saugöffnung, insbesondere einen Schlitz, aufweist.

20. Speichelsauger nach einem der vorhergehenden Ansprüche, **dadurch gekennzeichnet, dass** der Zungenschild ein Fimscharnier zur Ausbildung eines Gelenks mit einer Gelenkachse entlang der Längserstreckung des Zungenschildes aufweist.

21. Speichelsauger nach einem der vorhergehenden Ansprüche, **dadurch gekennzeichnet, dass** der Weichkörper von dem Formteil trennbar und auswechselbar ist, und insbesondere die Kombination aus Saugschlauch und Formteil autoklavierbar ist.
